# EUROPEAN PATENT APPLICATION

(11) **EP 1 410 808 A1**
(43) Date of publication of application: **21.04.2004**
(21) Application number: 02741373.1
(22) Date of filing: 01.07.2002
(51) Int. Cl.: A61K 45/00, A61K 31/551, A61P 27/02, A61P 27/06

(54) **OPTIC NERVE PROTECTING AGENTS CONTAINING ALPHA 1 RECEPTOR BLOCKER AS THE ACTIVE INGREDIENT**

(30) Priority: 02.07.2001 JP 2001201015
(71) Applicant: SANTEN PHARMACEUTICAL CO., LTD., Higashiyodogawa-ku, Osaka-shi, Osaka 533-8651 (JP); Eisai Co., Ltd., Tokyo 112-0002 (JP)
(72) Inventor: MIYAWAKI, N., Santen Pharmaceuitcal Co., Ltd., Ikoma-shi, Nara 630-0101 (JP); HARA, H., Santen Pharmaceutical Co., Ltd.,Kenkyuso, Ikoma-shi, Nara 630-0101 (JP); GOTO, W, Santen Pharmaceutical Co., Ltd, Kenkyusho, Ikoma-shi, Nara 630-0101 (JP)
(74) Representative: Peaucelle, Chantal
(86) International application number: PCT/JP2002/006627
(87) International publication number: WO 2003/004058

(57) **Abstract**

A novel pharmacological effect of an α₁ receptor blocker is found out. As the results of studies on the novel pharmacological effect of the α₁ receptor blocker, it is clarified that this blocker has an effect of protecting optic nerve. Thus, the α₁ receptor blocker is expected as being useful in optic nerve protecting agents to be used in treating retinal disease typified by normal tension glaucoma, retinal blood vessel occlusion, diabetic retinophathy ischemic optic neuropathy, macular degeneration, retinitis pigmentosa and Leber's disease.

## Description

### Technical Field

The present invention relates to an optic nerve protecting agent containing an α₁ receptor blocker as an active ingredient.

### Background Art

Human optic nerve is composed of about 1,000,000 optic nerve fibers. It is said that when 30 % of the fibers is disordered, abnormality can be detected with a static perimeter and that just when 50 % of the fibers is disordered, the influence can emerge on their visual field and be detected with a kinetic perimeter. Optic nerve fibers are composed of the axons of retinal ganglion cells and elongate to the lateral geniculate nucleus. Anatomically and physiologically, retinal ganglion cells can be classified into several types. Axons elongating from these types of retinal ganglion cells have individually different morphologies and functions. The disorder of optic nerve occurs, essentially due to a disorder of axonal transport. Axonal transport is disordered at the part of lamina cribrosa. The disorder of axonal transport means the death of optic nerve cells. It is suggested that apoptosis is involved in the death (Journal of the eye, 16(6) 833-841). When optic nerve cells are disordered, disorders of visual field and various retinal disorders occur, eventually leading to the possibility of visual loss. Therefore, the development of an optic nerve protecting agent for protecting optic nerve cells and preventing the death of the cells is desired.

Because α₁ receptor blockers bind to the adrenalin α₁ receptor to suppress neurotransmission, the α₁ receptor blockers are used as hypotensive agents. Bunazosin as an α₁ receptor blocker is commercially available as a hypotensive agent. Japanese Patent No. 2610619 reports that bunazosin has an action to reduce intraocular pressure and is useful for the treatment of high intraocular pressure. Further, JP-B-7-23302 reports a formulation for applying bunazosin to eyedrops. Further, it is reported that terazosin (WO 95/31200), prazosin (USP No. 4197301) and dapiprazole (JP-A-54-157576) as an α₁ receptor blocker have such action to reduce intraocular pressure.

Generally, it is considered that glaucoma is a disease caused by the rise of intraocular pressure, which induces organic disorders of optic nerve fibers at the site of the optic disc of optic nerve, involving the occurrence of the inversion of the optic disc and the defects of retinal nerve fibers to finally cause visual field disorders. In the field of research works over glaucoma, mainly, a method for suppressing the rise of intraocular pressure to indirectly prevent the disorders of visual field is studied.

It is expected that a drug capable of actively protecting optic nerve cells if found may directly prevent visual field disorders.

A disease involving a change of optic disc and the change of visual field has recently been drawing attention, although no rise of intraocular pressure is involved therein. The disease is called normal tension glaucoma. Normal tension glaucoma is a disease causing defects of visual field, due to the disorders of optic nerve, although the intraocular pressure is constantly within the normal range (at 21 mm Hg or lower). Even for the treatment of such normal tension glaucoma, it is needed to reduce intraocular pressure, and ocular hypotensive agents are administered. If a drug capable of protecting optic nerve can be found, the drug may be used for direct treatment or prevention of the disorders of visual field and may therefore be a more effictive drug. Therefore, the development of such drug with those effects is demanded.

For retinal diseases, additionally, retinal blood circulation disorders occupy a particularly important position among retinal diseases. The retinal blood circulation disorders cause the deficiency in the supply of oxygen and nutrients, so that retinal ganglion cells are dead. Typically, the symptoms of retinal blood circulation disorders include for example retinal blood vessel occlusion with occlusion or stenosis of retinal vein or retinal artery, diabetic retinopathy as one pathogenesis of retinal detachment, and ischemic optic neuropathy causing the occurrence of disorders of visual functions. It is suggested that the death of retinal ganglion cells is deeply involved in the onset or symptoms of macular degeneraten, retinitis pigmentosa and Leber's disease as other retinal diseases.

As has been described above, the protection of optic nerve cells enables the direct treatment or prevention of the disorders of visual field. Additionally, the protection thereof works as a useful measure for preventing or treating various retinal diseases.

As described above, α₁ receptor blockers have been know to have an action to reduce intraocular pressure. However, not any report exists about the effect of α₁ receptor blockers on the protection of optic nerve. Further, no report exists as to what kind of influences α₁ receptor blockers have on retinal diseases typically including for example normal tension glaucoma, retinal blood vessel occlusion, diabetic retinopathy, ischemic optic neuropathy, macular degeneraten, retinitis pigmentosa and Leber's disease.

As described above, the action of α₁ receptor blockers to protect optic nerve has not yet been known. Therefore, studies concerning the action of α₁ receptor blockers to protect the nerve have been a subject of much interest.

### Disclosure of the Invention

Therefore, the present inventors have made studies about the relation between α₁ receptor blockers and an optic nerve protecting action. Consequently, the inventors have found that α₁ receptor blockers have a great action to protect optic nerve cells.

The invention relates to an optic nerve protecting agent containing an α₁ receptor blocker as the active ingredient.

Additionally, the invention relates to a method for treating or preventing a disease due to the disorders of optic nerve cells, comprising administering an effective amount of an α₁ receptor blocker to a patient in need thereof, as well as use of an α₁ receptor blocker in the manufacture of a medicament to treat or prevent a disease due to disorders of optic nerve cells.

In the prevent invention, compounds with an action to block α₁ receptors include for example bunazosin, prazosin, terazosin, naftopidil, doxazosin, trimazosin, dapiprazole, alfuzosin, tamslosin, and KRG-3332.

Among the α₁ receptor blockers, α₁ receptor blockers having a basic heterocyclic ring in each chemical structure are preferable. The basic heterocyclic ring means a heterocyclic ring which has at least one nitrogen atom in the ring and which is basic. Saturated heterocyclic rings having two nitrogen atoms are more preferable among such basic heterocyclic rings. example imidazolidine, piperazine, and homopiperazine. The α₁ receptor blockers having a basic heterocyclic ring in each chemical structure include bunazosin, prazosin, terazosin, naftopidil, doxazosin, trimazosin and dapiprazole.

These drugs may take salt forms with inorganic acids and organic acids. Examples of the salts include for example hydrochlorides, sulfates, phosphates and oxalates.

In the course of the development and research of α₁ receptor blockers as therapeutic agents of glaucoma, unexpectedly, the inventors found that α₁ receptor blockers had an action to protect optic nerve cells. Thus, the prevent invention has been achieved. According to the method of Kashii et al. (IOVS. 1994, vol.35, no.2 685-695), the inventors had been studying their action on the death of rat fetal retinal nerve cells in culture as induced by glutamate addition and their action on the death of rat fetal retinal nerve cells in culture as induced by serum elimination. (The details are described in the following section of a pharmacological test.) The inventors consequently found that α₁ receptor blockers effectively suppressed the death of such nerve cells and would potentially be great drugs for protecting optic nerve.

In accordance with the invention, the α₁ receptor blockers may be administered orally or parenterally. The dosage forms thereof include for example tablets, capsules, granules, powders, eye drops and injections. Particularly, eye drops and injections are preferable. These can be formulated, using routine techniques.

Specifically, formulations described in for example JP-B-7-23302 and WO 95/31200 and commercially available formulations can be used.

Taking an example by eyedrops, formulation methods are described in detail. Using isotonic agents such as sodium chloride and concentrated glycerin, buffers such as boric acid, borax, sodium phosphate, and sodium acetate, surfactants such as polyoxyethylene sorbitan monooleate, stearate polyoxyl 40 and polyoxyethylene hardened castor oil, stabilizers such as sodium citrate and sodium edetate, and preservatives such as benzalkonium chloride and paraben depending on the necessity, such eyedrops can be prepared. The pH may be within a range acceptable for ophthalmic formulations. Preferred pH is within the range of 4 to 8. Eye ointment can be prepared, using usual base such as white Vaseline and fluid paraffin.

The dose may appropriately be selected, depending on the symptoms, the age and the like of patients. For oral administraten, the dose of 0.1 to 100 mg per day can be administered in one time or in several times. Eyedrops at 0.0001 to 1 w/v %, preferably 0.001 to 0.1 w/v % are instilled into eyes in one time or in several times per day.

The α₁ receptor blocker of the invention may be used in combination with other intraocular pressure reducing agents.

As an example, the results of a pharmacological test are shown below. The example is for higher understanding of the invention but never limits the scope of the invention.

### Best Mode for Carrying out the Invention

### Example

### [Pharmacological test]

So as to study the actions of α₁ receptor blockers for protecting optic nerve cells, the following actions were examined by the methods hereinbelow: (1) an action on the death of rat fetal retinal nerve cells in culture as induced by glutamate addition and (2) an action on the death of rat fetal retinal never cells in culture as induced by serum elimination.

### (1) The action of the α₁ receptor blocker on the death of rat fetal retinal nerve cells in culture as induced by glutamate addition

Retinal cells were taken out of a rat fetus (age of E18 days) and isolated, then cultured in an Eagle's basal culture medium containing 10 % bovine fetus serum on a polyethylene imine-coated plastic cover slip for 7 days (37 °C, 5 % CO₂/95 % air). On the eighth day and thereafter, the cells were cultured in an Eagle's basal culture medium containing 10 % horse serum. On the sixth day of the cultivation, cytosine arabinoside (10 µM) was added to suppress the growth of non-neuronal cells. On the eleventh day of the cultivation, the cells were cultured in a serum-free Eagle's basal culture medium supplemented with glutamate (1 mM) and an α₁ receptor blocker, for 10 minutes. Then, the cells were transferred to a serum-free Eagle's basic culture medium with no content of glutamate and an α₁ receptor blocker, and cultured for one hour. Subsequently, toxicity was determined by the trypan blue dye exclusion method. Specifically, the cells were stained with 1.5 % trypan blue solution for 10 minutes, fixed in 10 % neutral formalin solution and rinsed with physiological saline. Subsequently, the cells were counted with a phase contrast microscope. The cells stained with trypan blue were defined as dead cells, while the cells never stained were defined as live cells. Based on the rate of the live cells to the total cells (200 or more) , the survival rate of the culture cells was determined. A group with the addition of 1 mM glutamate alone and a group with the addition of both 1 mM glutamate and an α₁ receptor blocker were defined as "1 mM glutamate single-addition group" and "group of 1 mM glutamate + α₁ receptor blocker addition", respectively. These results were compared with the corresponding rate calculated from a "non-treated group" with no treatment. At this experiment, bunazosin hydrochloride, prazosin hydrochloride, terazosin hydrochloride and naftopidil hydrochloride were used as such α₁ receptor blockers. The results of the experiment are individually shown in Tables 1 to 4. Herein, average values are shown in the tables.

**Table 1**

| Effect of bunazosin hydrochloride | |
|---|---|
| | Survival rate (%) of retinal nerve cells in culture |
| Non-treated group (6) | 82.4 |
| 1 mM glutamate single-addition group (7) | 41.8 |
| Group of 1 mM glutamate + 10 µM bunazosin HCl addition (7) | 55.2 |
| Group of 1 mM glutamate + 100 µM bunazosin HCl addition (5) | 55.9 |

The numerical figure in () means the number of cases in each group.

**Table 2**

| Effect of prazosin hydrochloride | |
|---|---|
| | Survival rate (%) of retinal nerve cells in culture |
| Non-treated group (6) | 75.6 |
| 1 mM glutamate single-addition group (7) | 52.7 |
| Group of 1 mM glutamate + 1 µM prazosin HCl addition (7) | 65.2 |
| Group of 1 mM glutamate + 10 µM prazosin HCl addition (6) | 67.1 |

The numerical figure in () means the number of cases in each group.

**Table 3**

| Effect of terazosin hydrochloride | |
|---|---|
| | Survival rate (%) of retinal nerve cells in culture |
| Non-treated group (7) | 68.7 |
| 1 mM glutamate single-addition group (7) | 48.6 |
| Group of 1 mM glutamate + 0.01 µM terazosin HCl addition (7) | 69.8 |
| Group of 1 mM glutamate + 0.1 µM terazosin HCl addition (7) | 70.7 |

The numerical figure in () means the number of cases in each group.

**Table 4**

| Effect of naftopidil hydrochloride | |
|---|---|
| | Survival rate (%) of retinal nerve cells in culture |
| Non-treated group (7) | 69.9 |
| 1 mM glutamate single-addition group (7) | 47.9 |
| Group of 1 mM glutamate + 0.1 µM naftopidil HCl addition (7) | 59.2 |
| Group of 1 mM glutamate + 10 µM naftopidil HCl addition (7) | 68.9 |
| The numerical figure in () means the number of cases in each group. | |

### (2) The action of the α₁ receptor blocker on the death of rat fetal retinal nerve cells in culture as induced by serum elimination

Retinal cells were taken out of a rat fetus (age of E18 days) and isolated, then cultured in an Eagle's basal culture medium containing 10 % bovine fetus serum on a polylysine-coated glass cover slip for 7 days (37 °C, 5 % CO₂/95 % air). On the eighth day and thereafter, the cells were cultured in an Eagle's basal culture medium containing 10 % horse serum. On the sixth day of the cultivation, cytosine arabinoside (10 µM) was added to suppress the growth of non-neuronal cells. On the tenth day of the cultivation, the cells were cultured in a serum-free Eagle's basic culture medium or in a serum-free Eagle's basal culture medium supplemented with 10 µM bunazosin hydrochloride, for 24 hours. Subsequently, toxicity was determined by the trypan blue dye exclusion method. Specifically, the cells were stained with 1.5 % trypan blue solution for 10 minutes, fixed in 10 % neutral formalin solution and rinsed with physiological saline. Subsequently, the cells were counted with a phase contrast microscope. The cells stained with trypan blue were defined as dead cells, while the cells never stained were defined as live cells. Based on the rate of the live cells to the total cells (200 or more), the survival rate of the culture cells was determined. Using the TUNEL method, additionally, the TUNEL-positive cells (apoptosis cells) increasing due to the serum elimination and the TUNEL-negative cells (live cells) were counted, to calculate the rate of the TUNEL-positive cells to the total cells. A group cultured in the serum-free Eagle's culture medium and a group with the addition of the α₁ receptor blocker to the serum-free Eagle's culture medium were defined as "serum elimination group" and "group with serum elimination + α₁ receptor blocker addition", respectively. These results were compared with the corresponding rate calculated from a "non-treated group" with no treatment. Table 5 shows the results of the experiment using bunazosin hydrochloride as an α₁ receptor blocker. The numerical figures in the table express average values.

**Table 5**

| | Survival rate (%) of retinal nerve cells in culture | Rate (%) of TUNEL-positive cells |
|---|---|---|
| Non-treated group (5) | 79 | 6 |
| Serum elimination group (5) | 64 | 27 |
| Group with serum elimination + 10 µM bunazosin HCl addition (5) | 80 | 13 |

The numerical figure in () means the number of cases in each group.

As shown in the results shown in Tables 1 to 5, the α₁ receptor blockers have an effect of distinctly suppressing the death (apoptosis) of retinal nerve cells in culture. Thus, α₁ receptor blockers as an agent for protecting optic nerve cells such as retinal ganglion cells are useful as preventing or treating agents of retinal diseases typically including for example normal tension glaucoma, retinal blood vessel occlusion, diabetic retinopathy, ischemic optic neuropathy, macular degeneraten, retinitis pigmentosa, and Leber's disease.

### Industrial Applicability

The results of the pharmacological test indicate that α₁ receptor blockers such as bunazosin have an action for protecting optic nerve and are useful as therapeutic agents of retinal diseases typically including for example normal tension glaucoma, retinal blood vessel occlusion, diabetic retinopathy, ischemic optic neuropathy, macular degeneraten, retinitis pigmentosa, and Leber's disease. Thus, the invention provides an optic nerve protecting agent containing an α₁ receptor blocker as the active ingredient.

## Claims

1. An optic nerve cell protecting agent comprising an α₁ receptor blocker as an active ingredient.

2. The optic nerve cell protecting agent according to claim 1, wherein the α₁ receptor blocker is an α₁ receptor blocker having a basic heterocyclic ring in the chemical structure.

3. The optic nerve cell protecting agent according to claim 1, wherein the α₁ receptor blocker is bunazosin, prazosin, terazosin, naftopidil, doxazosin, trimazosin or dapiprazole.

4. The optic nerve cell protecting agent according to claim 1, wherein the optic nerve cell is a retinal ganglion cell.

5. The optic nerve cell protecting agent according to claim 1, the optic nerve cell protecting agent being used for the prevention or treatment of an ophthalmic disease.

6. The optic nerve cell protecting agent according to claim 5, wherein the ophthalmic disease is normal tension glaucoma.

7. The optic nerve cell protecting agent according to claim 5, wherein the ophthalmic disease is retinal blood vessel occlusion, diabetic retinopathy, ischemic optic neuropathy, macular degeneraten, retinitis pigmentosa or Leber's disease.

8. A treating or preventing agent of disorders of visual field comprising an α₁ receptor blocker and being based on the action for protecting optic nerve cells.

9. The treating or preventing agent of disorders of visual field according to claim 8, wherein the α₁ receptor blocker is an α₁ receptor blocker having a basic heterocyclic ring in the chemical structure.

10. The treating or preventing agent of disorders of visual field according to claim 8, wherein the α₁ receptor blocker is bunazosin, prazosin, terazosin, naftopidil, doxazosin, trimazosin or dapiprazole.

11. A method for treating or preventing a disease due to the disorders of an optic nerve cell, comprising administering an effective amount of an α₁ receptor blocker to a patient in need thereof.

12. The method for treating or preventing according to claim 11, wherein the α₁ receptor blocker is an α₁ receptor blocker having a basic heterocyclic ring in the chemical structure.

13. The method for treating or preventing according to claim 11, wherein the α₁ receptor blocker is bunazosin, prazosin, terazosin, naftopidil, doxazosin, trimazosin or dapiprazole.

14. The method for treating or preventing according to claim 11, wherein the optic nerve cell is a retinal ganglion cell.

15. The method for treating or preventing according to claim 11, wherein the disease is an ophthalmic disease.

16. The method for treating or preventing according to claim 15, wherein the ophthalmic disease is normal tension glaucoma.

17. The method for treating or preventing according to claim 15, wherein the ophthalmic disease is retinal blood vessel occlusion, diabetic retinopathy, ischemic optic neuropathy, macular degeneraten, retinitis pigmentosa or Leber's disease.

18. A method for treating or preventing disorders of visual field comprising administering an effective amount of an α₁ receptor blocker to a patient in need thereof, the method being based on the action for protecting an optic nerve cell.

19. The method for treating or preventing disorders of visual field according to claim 18, wherein the α₁ receptor blocker is an α₁ receptor blocker with a basic heterocyclic ring in the chemical structure.

20. The method for treating or preventing disorders of visual field according to claim 18, wherein the α₁ receptor blocker is bunazosin, prazosin, terazosin, naftopidil, doxazosin, trimazosin or dapiprazole.

21. Use of an α₁ receptor blocker in the manufacture of a medicament to treat or prevent a disease due to disorders of the protection of an optic nerve cell.

22. Use according to claim 21, wherein the α₁ receptor blocker is an α₁ receptor blocker having a basic heterocyclic ring in the chemical structure.

23. Use according to claim 21, wherein the α₁ receptor blocker is bunazosin, prazosin, terazosin, naftopidil, doxazosin, trimazosin or dapiprazole.

24. Use according to claim 21, wherein the optic nerve cell is a retinal ganglion cell.

25. Use according to claim 21, wherein the disease is an ophthalmic disease.

26. Use according to claim 25, wherein the ophthalmic disease is normal tension glaucoma.

27. Use according to claim 25, wherein the ophthalmic disease is retinal blood vessel occlusion, diabetic retinopathy, ischemic optic neuropathy, macular degeneraten, retinitis pigmentosa or Leber's disease.

28. Use of an α₁ receptor blocker in the manufacture of a medicament to treat or prevent disorders of visual field, the medicament being based on the action for protecting an optic nerve cell.

29. Use according to claim 28, wherein the α₁ receptor blocker is an α₁ receptor blocker having a basic heterocyclic ring in the chemical structure.

30. Use according to claim 28, wherein the α₁ receptor blocker is bunazosin, prazosin, terazosin, naftopidil, doxazosin, trimazosin or dapiprazole.
